(19)

Europäisches
Patentamt
European
Patent Office
Office européen
des brevets

(11) **EP 4 385 417 A1**

(12) **EUROPEAN PATENT APPLICATION**

(43) Date of publication:
**19.06.2024 Bulletin 2024/25**

(21) Application number: **22214097.2**

(22) Date of filing: **16.12.2022**

(51) International Patent Classification (IPC):
**A61B 6/03** (2006.01)    **A61B 6/00** (2024.01)

(52) Cooperative Patent Classification (CPC):
**A61B 6/4021; A61B 6/032; A61B 6/4007;
A61B 6/4028; A61B 6/486; A61B 6/5258;
A61B 6/547; A61B 6/583; A61B 6/586; A61B 6/587**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventor: **KOEHLER, Thomas
Eindhoven (NL)**

(74) Representative: **Philips Intellectual Property &
Standards
High Tech Campus 52
5656 AG Eindhoven (NL)**

(54) **CALIBRATION METHOD FOR USE IN CT IMAGING**

(57)    A method for calibration within the context of a CT imaging mechanism in which projection data is acquired over a series of angular positions of a CT scanner about a body, and wherein at least two angularly displaced x-ray sources (e.g. x-ray tube focal spots) are alternately activated as the scanner rotates orbitally around the body. The alternately activated sources may be focal spots or may be other sources. It is proposed to detect a spatial transformation (i.e. a spatial shift) in the whole or part of the 2D attenuation pattern measured from consecutive first and second projections, corresponding respectively to the first x-ray source and second x-ray source. The concept is to use this shift in the measured projection patterns to infer the separation between the x-ray sources. It can be determined whether the separation is within allowable operational constraints.

FIG. 5

**Description**

FIELD OF THE INVENTION

The present invention relates to the field of CT imaging, particularly to alternating dual source CT imaging, such as dual focal-spot imaging.

BACKGROUND OF THE INVENTION

In state-of-the-art medical CT systems, dual-focal spot (DFS) acquisition is commonly used to increase spatial resolution and reduce aliasing. In dual focal spot acquisition, the location of the x-ray tube focal spot (i.e. the x-ray source location) is alternated at high frequency between two adjacent positions throughout the orbital rotation of the CT scanner about the examination region. Often, the source is configured so that the focus jumps between subsequent detector readings by $\pm 0.25$ times the radial sampling interval of the detector, back and forth around the geometrical center of the system. Additionally, there may be also a jump in z direction. In effect, two datasets are acquired concurrently as the x-ray scanning apparatus rotates about the 3D object, one corresponding to the first focal spot, and one corresponding to the second focal spot.

The dual focal spot (DFS) acquisition approach is described in detail in the paper: M. Kachelriess et al., Flying Focal Spot (FFS) in Cone-Beam CT, IEEE TNS, 53(3), 2006.

The dual focal spot (DFS) acquisition approach is also described in the document US4637040.

System reliability is an important consideration in modem CT systems. However, CT systems are also very complex machines and occasional down time is unavoidable. Therefore, it is desirable to build automatic means for fault detection and fault prediction in order to alert users to potential errors which might result in imaging, to minimize system down time by proactively triggering visits by a field service engineer, and to provide accurate diagnostic information to allow for a quick identification of the root cause of system problems.

One part of a CT system that requires accurate calibration is the x-ray source positioning.

Within the context of dual focal spot imaging, if the accuracy of the focal spot positions changes over time and deviates from the desired values, image quality degrades. Specifically, spatial resolution will degrade, and aliasing artefacts may result. However, de-calibration of x-ray tube focal spots tends to occur gradually, so that it may not be noticed in degraded image quality until artefacts are already significant.

It is noted that although this problem is presented with reference to dual focal spot acquisition, the same problem would apply for other types of dual-source CT imaging, including those which might not use two focal spots on an x-ray tube, but a different technical means for implementing the alternating sources.

SUMMARY OF THE INVENTION

The invention is defined by the claims.

According to examples in accordance with an aspect of the invention, there is provided a computer-implemented method for determining calibration status of x-ray point sources within a dual point source computed tomography (CT) acquisition apparatus, for example a dual focal spot acquisition apparatus.

The method comprises receiving dual point source CT data comprising x-ray detector measurement data for each of a series of x-ray projections, the measurement data corresponding to measurement data acquired with an x-ray generator of a CT acquisition apparatus providing alternately activated first and second x-ray sources, for example corresponding to first and second x-ray tube focal spots. The sources are displaced relative to one another along an orbital rotational axis of the generator within the CT acquisition apparatus. The measurement data includes for each x-ray projection a 2D attenuation pattern corresponding to measured attenuation values for a 2D array of detector elements.

The method comprises performing a point source calibration check, comprising: determining, using the measurement data, a shift of the whole or part of the attenuation pattern of a first and second consecutive projection, each of the first and second projection generated using a different one of the two point sources. The method preferably further comprises determining whether the shift is within a pre-defined expected range. For example, the expected range may correspond to an expected range given an expected distance or displacement between the two x-ray point sources (within the x-ray generator).

Thus, embodiments of the present invention propose to check the calibration of the dual point sources, in particular to check a separation distance or displacement between the point sources. The inventor proposes to do this by detecting a spatial transformation between the whole of, or a part of, or a point within the 2D attenuation pattern which is measured by the detector with the first point source and the second point source. This shift in the measured attenuation pattern maps onto the separation between the point sources and thus provides a measure by which the target parameter of the point source separation can be probed.

The first and second x-ray point sources are displaced relative to one another within a reference frame of the x-ray generator, i.e. they are displaced relative to one another on or in the x-ray generator. Their displacement is preferably a displacement along the direction of the orbital axis of the gantry. Thus, the generator carries an x-ray generating arrangement operable to generate two point sources displaced relative to one another, and wherein the orbital rotation of the generator moves each of these point sources around a series of orbital angular positions to acquire the first and second measurement data.

The x-ray generator may comprise at least one x-ray tube.

The first and second point-sources may typically correspond to first and second x-ray tube focal spots within a dual focal spot CT acquisition apparatus. Alternatively, the method is also applicable to any other type of dual point source system.

Although reference is made above to an attenuation pattern, equally this could be an intensity pattern representing the radiation reaching the detector (i.e. the attenuation is the logarithm of the expected zero-attenuation intensity, given the source intensity and the system geometry, minus the logarithm of the measured intensity). Intensity and attenuation are related by Beer's law, and thus are effectively interchangeable. Thus, in this disclosure, reference to a 2D attenuation pattern could be understood equally as corresponding to intensity, without any change in the inventive principles. The attenuation pattern measured from one particular source position is also termed a 'projection'.

The reference above to a shift in the whole or part of the attenuation pattern means for example a spatial shift, i.e. a transformation, e.g. a translation or a rotation. Thus, in some embodiments, the determining of the shift may comprise determining a transformation, e.g. rotation or translation, of the whole or part of the attenuation pattern of a first and second consecutive projection.

In some embodiments, the determining the shift comprises determining a translation of the whole or part of the attenuation pattern in said direction of the orbital rotation axis of the generator.

In some embodiments, the determining the shift comprises identifying a characteristic (spatial) point in the respective 2D attenuation patterns of the first and second projections and determining a translation in said characteristic point between the first and second projections. By using a characteristic point, this makes the analysis easier, since it is only necessary to track the shift in the single point rather than the whole projection.

In some embodiments, the characteristic point may be defined in each 2D attenuation pattern based on a function of attenuation values in the attenuation pattern, weighted by attenuation value position (meaning the position of the corresponding detector element) in the 2D pattern.

In some embodiments, the characteristic point may be defined as a center of attenuation along at least one dimension of each 2D attenuation pattern, the center of attenuation being the attenuation analog of center of mass.

By way of example, the center of attenuation along at least one dimension of the attenuation pattern may be defined by the equation:

$$c = \frac{\sum_j j * p'(j)}{\sum_j p'(j)}$$

where $p'(j) = \Sigma_i p(i,j)$,
where $i$ is an index of the detector element row and $j$ is an index of the detector element column, and where $p(i,j)$ is the attenuation measurement value for detector element in row $i$, column $j$.

Thus, it can be seen that this mirrors the equation for center of mass, but with mass replaced by attenuation measured at a given detector pixel, and with the spatial co-ordinate provided by the row index j.

Additionally or alternatively to the approach of detecting a shift of a characteristic point in the attenuation patterns, another approach is to perform a registration between at least a patch of each of the first and second projections.

This, in some embodiments, the determining the shift comprises identifying a characteristic sub-region of the 2D attenuation pattern of each of the first and second projections, and performing a translational registration between the characteristic sub-regions of the two projections.

Here the idea is to detect a translation by analytical translational registration of small patches of two consecutive projections. Methods for doing this are known, and examples will be described in the detailed description.

In some embodiments, the method further comprises re-binning the measurement data for each of the projections into parallel beam geometry prior to performing before the calibration check. In preferred embodiments, a wedge-beam rebinning is performed. Wedge beam rebinning is described for example in section II.A of the paper: Shechter et al.: The frequency split method for helical cone-beam reconstruction. Med Phys. 2004 Aug;31(8):2230-6.

In some embodiments, the method may comprise, in advance of the calibration check, performing, for each projection, an imaged object alignment check. In particular, the accuracy of the calibration check is improved if the first and second projections are non-truncated, meaning that each projection fully contains the target imaged object.

To achieve this, in some embodiments, the imaged object alignment check may comprise first identifying measured attenuation values in the 2D attenuation pattern corresponding to a pre-defined boundary region of the array of detector elements. The alignment check may further comprise determining whether each of the measured attenuation values in the boundary region fall within a pre-defined null range, the null range corresponding to a zero-attenuation measurement. The alignment check may further comprise, responsive to determining that not all of the measured attenuation values in the boundary region fall within the null range, generating an alert message and/or aborting the calibration check.

Additionally or alternatively to the approaches already outlined above, in some embodiments, the determining the shift comprises using a trained machine learning algorithm which is trained to receive as input the measurement data for the first and second projections and to generate as output a value of the shift between the 2D attenuation patterns of the first and second projections.

In some embodiments, the determining the shift in the whole or part of the attenuation patten of the first and second projection is performed indirectly based on a detection operation performed in the image domain.

For example, in some embodiments, the determining the shift in the whole or part of the attenuation patten of the first and second projection is performed by: reconstructing a first image using only the x-ray projections among the received series of x-ray projections acquired with the first of the x-ray point sources; reconstructing a second image using only the x-ray projections among the received series of x-ray projections acquired with the second of the x-ray point sources; and detecting a rotation between the first image and the second image. In particular, the first and second images may be axial images, meaning images in the plane of rotation of the scanner, normal to the z-axis.

In some embodiments, the method may comprise computing an actual separation or spacing or displacement between the first and second x-ray point sources based on a determined translation or rotation in the whole or part of the attenuation pattern between the first and second consecutive projections. In some embodiments, this may be a spatial distance between the sources. In some embodiments, it may be an angular separation/displacement between the point sources.

In some embodiments, the method comprises determining a deviation in the distance between the first and second x-ray point sources and a target distance between the first and second x-ray point sources. The method may further comprise communicating with an image reconstruction module to apply an additional correction step to a reconstruction algorithm comprised by the reconstruction module based on the determined deviation.

In some embodiments, the method may further comprise supplying the CT data to the reconstruction module and applying the reconstruction algorithm with the additional correction step to the CT data.

In some embodiments, the method further comprises generating a status report indicative of a difference between the computed separation or spacing or displacement (e.g. distance) between the first and second x-ray point sources and a pre-defined target separation or spacing or displacement between the first and second x-ray point sources, and transmitting the status report to a server computer. For example, this could be a computer of a maintenance or servicing team.

In some embodiments, the method further comprises storing the measured separation or spacing or displacement (e.g. distance) between the first and second x-ray point sources in a data log.

In some embodiments, the method comprises generating an alert signal for communication to a user interface or a further computing device responsive to said determined shift being outside of said expected range.

In another aspect, there is provided a computer-implemented method for reconstructing CT image data from a dual point source computed tomography (CT) acquisition apparatus having acquired measurement data with an x-ray generator providing alternately activated first and second x-ray point sources, the point sources displaced relative to one another along an orbital rotational axis of the generator within the CT acquisition apparatus, and wherein the measurement data includes for each x-ray projection a 2D attenuation pattern corresponding to measured attenuation values for a 2D array of detector elements, the method comprising obtaining a distance estimate between the first and second x-ray point sources; and reconstructing CT image data based on the distance estimate.

Another aspect of the invention is a computer program product comprising computer program code configured, when run on a processor, to cause the processor to perform a method in accordance with any embodiment described in this document or in accordance with any claim in the application.

Another aspect of the invention is a processing device comprising: an input/output; and one or more processors configured to perform a method. The method comprises: receiving, at the input/output, dual point source CT data comprising x-ray detector measurement data for each of a series of x-ray projections, the measurement data corresponding to measurement data acquired with an x-ray generator of a CT acquisition apparatus providing alternately activated first and second x-ray point sources, for example corresponding to first and second x-ray tube focal spots, the point sources displaced relative to one another along an orbital rotational axis of the generator within the CT acquisition apparatus, and wherein the measurement data includes for each x-ray projection a 2D attenuation pattern corresponding to measured attenuation values for a 2D array of detector elements. The method further comprises performing a point source calibration check, comprising: determining, using the measurement data, a relative shift between the whole or part of the attenuation pattern of a first and second consecutive projection, each of the first and second projection generated using a different one of the two point sources. The method may further comprise determining whether the shift is within a pre-defined expected range. The pre-defined expected range may for example correspond to an expected range given an expected distance between the two x-ray point sources.

Another aspect of the invention is a system, comprising: a dual point source CT acquisition apparatus; and a processing device as outlined above or in accordance with any embodiment described in this document, or in accordance with any claim of the application.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 schematically illustrates an example CT acquisition apparatus in accordance with one or more embodiments;

Fig. 2 schematically illustrates a dual focal spot imaging protocol according to a state-of-the-art method;

Fig. 3 schematically illustrates a fan of x-ray beams generated by a single x-ray focal spot source (single x-ray point source), in accordance with a state-of-the-art CT scanning method;

Fig. 4 schematically illustrates a fan of x-ray beams generated in accordance with a dual focal spot CT scanning operation;

Fig. 5 outlines steps of an example method in accordance with one or more embodiments of the invention;

Fig. 6 schematically outlines components and a processing flow of an example processing arrangement in accordance with one or more embodiments of the invention;

Fig. 7 illustrates a 2D attenuation pattern captured by a 2D detector for one example projection;

Fig. 8 illustrates a plot of a computed shift in a characteristic point of first and second projections over a series of consecutive frames during a scan;

Fig. 9 illustrates registration of a selected patch of two consecutive projections for detecting a shift in selected patch;

Fig. 10 schematically illustrates sampling positions of x-ray point sources in a calibrated and uncalibrated scenario;

Fig. 11 provides a further illustration of the geometry of the CT apparatus;

Figs. 12 illustrates the presence of a rotation in the image domain caused by the drift in the x-ray point source separation;

Fig. 13 illustrates an absence of a rotation in the image domain in the case of well-calibrated dual x-ray sources;

Fig. 14 illustrates the process of checking that a projection is non-truncated through a check for zero attenuation at the lateral detector areas; and

Fig. 15 illustrates detector measurements for projections in r, $\phi$ space; and

Fig. 16 illustrates the mapping of measured rays into r, $\phi$ space.

DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs are merely schematic and are not drawn to scale. It should also be understood that the same reference numerals are used throughout the Figs to indicate the same or similar parts.

The invention provides a method for calibration within the context of a CT imaging mechanism in which projection data is acquired over a series of angular positions of a CT scanner about a body, and wherein at least two angularly displaced x-ray sources (e.g. x-ray tube focal spots) are alternately activated as the scanner rotates orbitally around the body. The alternately activated sources may be focal spots or may be other sources. It is proposed to detect a spatial transformation (e.g. a spatial shift) in the whole or part of the 2D attenuation pattern measured from consecutive first and second projections, corresponding respectively to the first x-ray source and second x-ray source. The concept is to use this shift in the measured projection patterns between the two projections to infer the separation between the x-ray sources. It can be determined whether the separation is within allowable operational constraints.

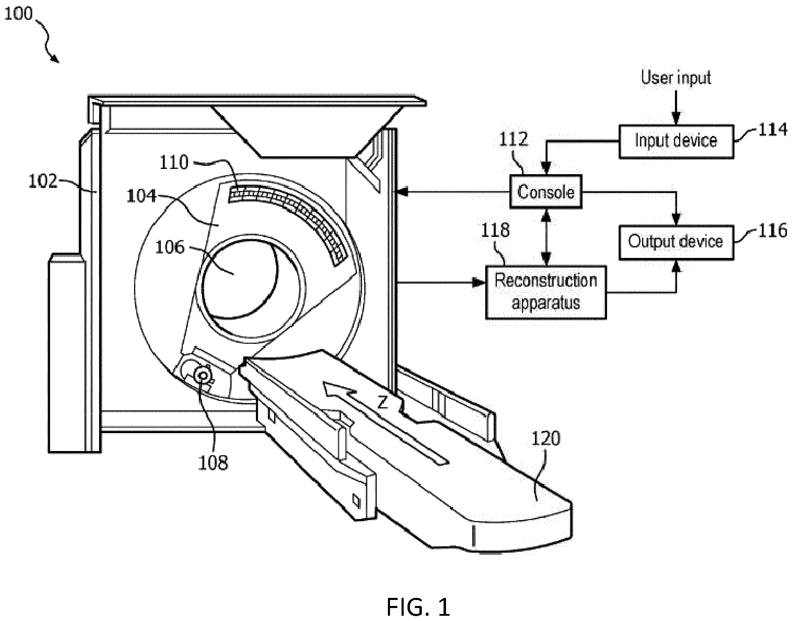

To aid understanding of forthcoming descriptions, Fig. 1 illustrates an example computed tomography (CT) imaging system 100, such as an x-ray CT scanner.

The imaging system 100 includes a generally stationary gantry 102 and a rotating gantry 104. The rotating gantry 104 is rotatably supported by the stationary gantry 102 and rotates orbitally around a longitudinal or z-axis, extending axially through an examination region 106 at a central annulus of the gantry.

A patient support 120, such as a couch, supports an object or subject such as a human patient in the examination region. The support 120 is configured to move the object or subject for loading, scanning, and/or unloading the object or subject.

A radiation source or generator 108, such as an x-ray tube, is rotatably supported by the rotating gantry 104. The radiation generator 108 rotates with the rotating gantry 104 and emits radiation that traverses the examination region 106.

A radiation sensitive detector array 110 subtends an angular arc opposite the radiation generator 108 across the examination region 106. The detector array 110 includes one or more rows of detectors that extend along the z-axis direction, detects radiation traversing the examination region 106, and generates projection data indicative thereof.

For cone-beam scanning, the radiation source generates an x-ray cone beam, and the detector array is a 2D detector array. The rotation of the radiation generator and detector around the examination region 106 (which receives a body to be scanned) may be described as orbital rotation, with the trajectory of rotation described as the orbital angular rotation axis or path.

A general-purpose computing system or computer may serve as an operator console 112 and may include an input device(s) 114 such as a mouse, a keyboard, and/or the like and an output device(s) 116 such as a display monitor, a filmer or the like. The console 112 allows an operator to control operation of the system 100.

A reconstruction apparatus 118 processes the projection data and reconstructs image data, for example volumetric image data. The data can be displayed through one or more display monitors of the output device(s) 116.

The reconstruction apparatus 118 in conventional systems may employ a filtered-backprojection (FBP) reconstruction, a (image domain and/or projection domain) reduced noise reconstruction algorithm (e.g., an iterative reconstruction) and/or other algorithm. It is to be appreciated that the reconstruction apparatus 118 can be implemented through a microprocessor(s), which executes a computer readable instruction(s) encoded or embed on computer readable storage medium such as physical memory and other non-transitory medium. Additionally or alternatively, the microprocessor(s) can execute a computer readable instruction(s) carried by a carrier wave, a signal and other transitory (or non-transitory) medium.

As briefly discussed above, to increase spatial resolution and sampling, the dual focal spot (DFS) acquisition approach has been developed. In dual focal spot acquisition, the location of the x-ray tube focal spot (i.e. the x-ray point source location) is alternated at high frequency between two adjacent positions throughout the orbital rotation of the CT scanner. In effect, two datasets are acquired concurrently as the x-ray scanning apparatus rotates about the 3D object, one corresponding to the first focal spot position, and one corresponding to the second focal spot position. Each focal spot forms an effective x-ray point source. The terms 'focal spot' and 'point source' may be used interchangeably in this disclosure.

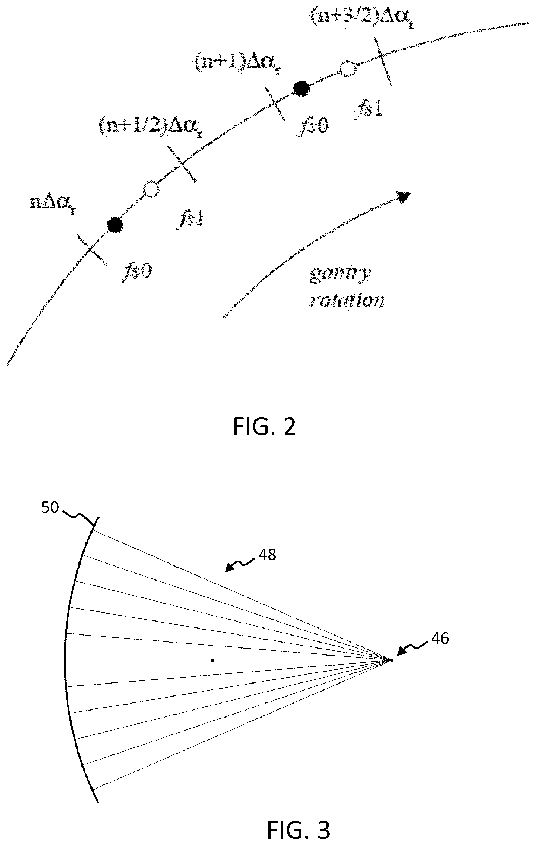

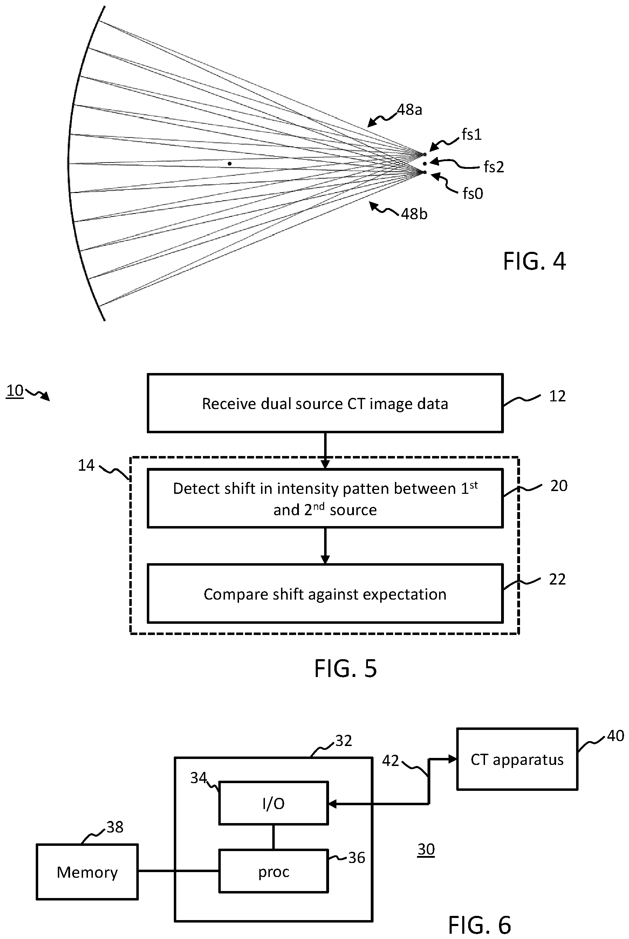

This is illustrated schematically in Fig. 2. This illustrates a portion of a rotation arc of the x-ray generator about the body being scanned. The x-ray focal spot positions, fs0, fs1 for each measured sample are shown as solid and open circles, respectively. Measurement samples for the two focal spots are acquired sequentially at each of a series of regular angular intervals, $\Delta\alpha_r$. The regular angular interval $\Delta\alpha_r$ is commonly referred to in the art as the 'angular sampling' interval of the system. To clarify further, for each pair of the two focal spots, there is a geometric center point or midpoint between the two. This midpoint is labelled as fs2 in Fig. 4. The system is controlled so that these center or midpoints are at equidistant angular intervals $\Delta\alpha_r$ along the rotation path. In other words, since the entire gantry moves at constant speed around the patient, the position of the center-points between each pair of focal points are equi-angularly distributed along the rotation path.

The two x-ray focal spots are preferably at a fixed distance relative to one another relative to the x-ray generator, e.g. relative to a tube housing of an x-ray tube of the generator. The reference in this document to "point sources displaced relative to one another along an orbital rotational axis of the generator within the CT acquisition apparatus" may be understood as referring for example to this distance between fs0 and fs1.

It is also noted that, for a constant rotation speed and a constant sampling rate of the detector (e.g., one detector readout every x degrees of gantry rotation), then the series of focal spot positions is not equi-angular (as is apparent from Fig. 2 for example). However, the respective center points between each pair of focal spot positions fs0, fs1 are equiangularly distributed.

There are different ways to create the alternating x-ray point sources. Both may be provided by a single x-ray tube having a pair of filaments. Alternatively, they may be provided by a radiation source having a pair of x-ray tubes, each tube including a distinct point source of radiation. Preferably, however, they may be provided by deflection means, e.g. electrostatic or electromagnetic, for deflecting an electron beam between at least two distinct focal spots on a target electrode.

The fan of x-ray beams 48 produced by a single focal spot 46 is shown schematically in Fig. 3, where the plane of the 2D detector is illustrated by arc 50.

By way of comparison, Fig. 4 schematically illustrates the respective fans of x-ray beams 48a, 48b produced by each of a pair of focal spots fs0, fs 1 respectively.

Embodiments of the present invention are based on performing a calibration check to determine whether the separation distance between the two focal spots is as expected. The basic approach proposed for achieving this is to determine a spatial shift or transformation of the whole or part of the attenuation pattern detected for a first and second consecutive projection acquired by the CT system, wherein the first projection is acquired with the first focal spot (fs0) and the second projection is acquired with the second focal spot (fs1). In other words, it is proposed to compare consecutive projections acquired from the first and second focal spots and detect a translation between them (or a portion of, or section of, or point within) them and use this as an indicator of the separation between the focal spots. Either this shift parameter can be directly assessed as a proxy for the separation between the focal spots or the shift parameter can be mapped to an estimate for the true separation between the focal spots.

One example approach is to identify a characteristic point in the respective 2D attenuation patterns of the first and second projections and determine a shift or transformation in said characteristic point between the first and second projections. For example, in a preferred approach, the characteristic point is a center-of-attenuation point, by which is meant the analog of center-of-mass for attenuation. Another alternative characteristic point is to use the maximum attenuation point of the projection or maximum attenuation point within a pre-selected patch of the projection.

Another example approach is to perform spatial registration between at least a portion of the first and second projections, e.g. between a selected spatial patch of each of the first and second projections. This could be a translational registration or a rotational registration for example.

Another example approach is to use a trained machine learning algorithm to detect the shift.

Fig. 5 outlines in block diagram form steps of an example method 10 according to one or more embodiments of the present invention. The steps will be recited in summary, before being explained further in the form of example embodiments.

The method 10 may be implemented by a computer or processor.

The method is for determining a calibration status of dual point sources of an x-ray generator within a dual point source computed tomography (CT) acquisition apparatus (e.g. a dual focal spot acquisition apparatus).

The method 10 comprises receiving 12 dual point source CT data comprising x-ray detector measurement data for each of a series of x-ray projections. The measurement data corresponds to measurement data acquired with an x-ray generator of a CT acquisition apparatus providing alternately activated first and second x-ray point sources, for example corresponding to first and second x-ray tube focal spots. The point sources are displaced relative to one another along an orbital rotational axis of the generator within the CT acquisition apparatus. The measurement data includes for each x-ray projection a 2D attenuation pattern corresponding to measured x-ray attenuation values (or, alternatively, measured x-ray intensity values) for a 2D array of detector elements.

The method further comprises performing a point source calibration check 14, for example a focal spot calibration check. The calibration check 14 comprises determining 20, using the measurement data, a shift of the whole or part of the attenuation pattern between a first and second consecutive projection, each of the first and second projection generated using a different one of the two point sources. The calibration check may further comprise determining 22 whether the shift is within a pre-defined expected range (i.e. comparing the shift against an expectation). For example, the expected range may correspond to an expected range given an expected separation or distance between the two x-ray point sources.

The method may further comprise generating a data output based on a result of the calibration check, e.g. an indication as to whether the shift is within the expected range.

As noted above, the method can also be embodied in hardware form, for example in the form of a processing unit which is configured to carry out a method in accordance with any example or embodiment described in this document, or in accordance with any claim of this application.

To further aid understanding, Fig. 6 presents a schematic representation of an example processing device 32 configured to execute a method in accordance with one or more embodiments of the invention. The processing device is shown in the context of a system 30. The processing device 32 alone can be provided as an aspect of the invention, and the system also forms another aspect of the invention. The system need not comprise all of the components shown but can comprise just a subset of them.

The processing device 32 comprises an input/output 34 or communication interface and one or more processors 36. The one or more processors 36 are configured to perform a method in accordance with any example or embodiment described in this document, or in accordance with any claim of this application.

The system 30 in this example further comprises a CT acquisition apparatus/system 40, e.g. a dual point source CT acquisition apparatus, e.g. a dual focal spot (DFS) acquisition apparatus. The CT apparatus generates dual point source CT data 42 which is received at the input/output of the processing device 32.

As mentioned above, the invention can also be embodied in software form. Thus, another aspect of the invention is a computer program product comprising computer program instructions or code configured when run by a processing device 32 comprising one or more processors 36 to cause the processing device to perform a method in accordance with any embodiment described in this disclosure or in accordance with any claim of this application. Optionally, the system 30 or the processing device 32 may further comprise a memory 38 which stores computer program instructions configured to cause the one or more processors 36 of the processing device 32 described above to perform a method in accordance with any embodiment of the invention. The memory 38 might also store one or more registers for use in the method. By way of example, the memory 38 might store a value for said pre-defined expected range.

Implementation details for one or more example embodiments will now be outlined in more detail.

As discussed above, detecting a potential miscalibration in the x-ray focal spots is valuable since this allows for remedial servicing work to be requested or queued for action and avoids continuing to acquire imaging data which may contain artefacts which might skew the results. Embodiments can be performed for example as an on-the-fly check applied to acquired measurement data from the CT acquisition system.

Additionally, in some embodiments, an adjustment can be made to the projection data or an adjustment can be made to the reconstruction algorithm to compensate for any detected miscalibration of the focal spots. This might for example be applied as a temporary interim measure while servicing is awaited.

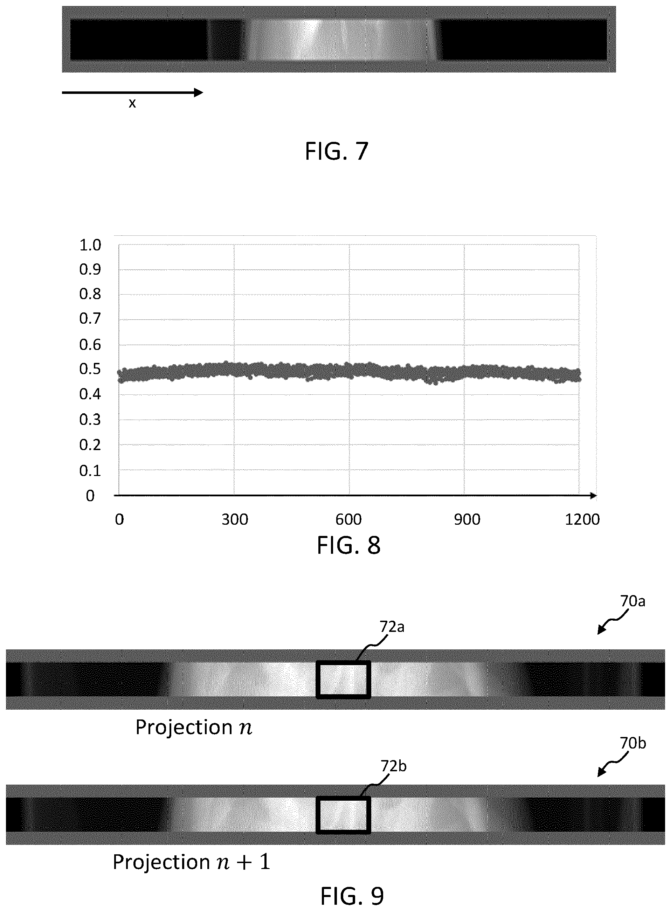

Reference is made to Fig. 7 which shows an example 2D attenuation pattern for a single x-ray projection acquired with a single one of two x-ray point sources of an x-ray generator. The 2D attenuation pattern corresponds to measured attenuation values for a 2D array detector (meaning a detector having a 2D array of detector elements). Embodiments of the invention propose to acquire at least a first and second such attenuation pattern for a first and second consecutive projection, one corresponding to the first x-ray point source and one corresponding to the second x-ray point source, and to detect a shift of the whole or part of the attenuation patterns of said first and second consecutive projections.

When two such consecutive projections are compared to one another, the result has the appearance of a slight shift of the imaged object, for example from left to right. This shifting in the detector attenuation pattern can be used to infer a separation between the point sources.

In particular, according to at least one set of embodiments, it is proposed to determine the shift in the attenuation pattern between the first and second projection by determining a translation in said direction of the orbital rotation axis of the generator. For example, this corresponds to detecting a shift in the direction labelled 'x' the example detector attenuation pattern shown in Fig. 7. This is a direction for example of incrementing detector column, i.e. the direction of detector column index, j.

A particularly efficient approach to doing this is to identify a common characteristic point in the respective attenuation patterns of the first and second projections and then to detect a translation in said characteristic point between the first and second projections. In a very simple example, the characteristic point might be a maximum attenuation point (minimum intensity) or a minimum attenuation point (maximum intensity). Given the very short time interval between the first and second projections (assuming a high frequency of alternation between the first and second point sources), then it is a reasonable assumption that maximum or minimum attenuation point in each projection ought to correspond to a same physical path through the imaged object, and thus its shift can be taken to be reflective of the shift in position of the point source between the two projections.

A more robust approach is to identify a characteristic point which is defined in each 2D attenuation pattern based on a function of attenuation values in the attenuation pattern, weighted by intensity value position (meaning detector element position) in the 2D pattern.

A simple and powerful example is to identify a characteristic point in each projection attenuation pattern which is a 'center of attenuation' point. By this is meant a point which is the analog of center of mass but for intensity or attenuation (depending upon which of these the projection pattern represents).

This is based on the realization by the inventors that that the jump in the x-ray source position leads to a good approximation of this shift of the "center of gravity" of the projection.

What is meant here is that the characteristic point can be defined as a center of attenuation along at least one dimension of each 2D attenuation pattern, the center of attenuation being the intensity analog of center of mass.

In particular, a mathematical expression for the center of attenuation along at least one dimension of the attenuation pattern can be derived as follows.

Let $p(i,j)$ be the projection values, with $i$ being the detector row and $j$ the column. Of interest is the center of attenuation in the $j$-direction only, so a first step is to take an average over the rows:

$$p'(j) = \sum_i p(i,j)$$

A next step is to calculate the center of attenuation as:

$$c = \frac{\sum_j j * p'(j)}{\sum_j p'(j)}$$

This is hence the analog of center of mass but for attenuation.

The result of this is a value in units of the detector column index, $j$, which corresponds to the center of attenuation of the projection. It might be a non-integer number but this does not matter for purposes of comparison of the value between the first and second projection.

This center of attenuation value can be calculated for the first and second projections and the values compared to determine a translation along the direction of the detector column index, $j$.

Thus, by calculation of the center of attenuation of each projection and calculation of the difference values of subsequent readings, it can be measured if the focal spot positions are as they should be. In other words, the translation in the center of attenuation between the first and second projections can be compared to a pre-defined expectation range to see whether it falls within this range.

By way of one example, it can be checked whether the focal spot separation is in fact within a pre-defined range of a target multiple of the angular sampling interval, $\Delta\alpha_r$.

Fig. 8 illustrates a plot of a computed shift in a center-of-attenuation point of first and second projections over a series of consecutive frames during a scan. In this example, the difference of the center of attenuation of the two readings within each frame of a dual focal spot acquisition is shown. The x-axis indicates frame number. The y-axis indicates the difference in the center-of-attenuation between the first and second projections, in units of the detector angular sampling interval, $\Delta\alpha_r$.

As this was a well-calibrated clinical system, the difference is very close to the target value (in this example, 0.5 times the detector angular sampling interval).

Additionally or alternatively to detecting a shift in a characteristic point, according to some embodiments, the separation between the point source positions can be performed based on detecting a registration between patches of the first and second projections (corresponding respectively to the first and second point sources).

In other words, in some embodiments, the determining the shift can comprise identifying a characteristic sub-region of the 2D attenuation pattern of each of the first and second projections, and performing a translational registration between the characteristic sub-regions of the two projections.

The registration can be performed analytically in some embodiments. An example implementation will now be explained.

Reference is made to Fig. 9.

The general principle is to select a region of interest in the first projection n and then to seek to determine (preferably with sub-pixel accuracy) where, in the subsequent (second) projection, $n+1$, this region is. Fig. 9 shows the measured detector patterns 70a, 70b for projection n and for projection $n+1$ respectively.

The algorithm for implementing the registration process might proceed with the following steps.

A first step is to select or identify a region of interest 72a in projection n. This might, by way of example, be a region of interest with maximum average intensity or attenuation.

A next step may be to up-sample the region of interest by a factor $m$ (e.g. using an interpolation method such as a cubic splines method).

A next step might be to up-sample projection n + 1 by the same factor $m$.

A next step may be to calculate the convolution of the up-sampled region of interest with the up-sampled projection n + 1 . In other words, up to a constant scaling, the correlation of the region of interest in the projection n with the projection $n+1$ is calculated.

A next step is to find the position within the convolution of the maximum value. Its position is an indicator how much the "template" structure defined as the region of interest 72a has moved in the subsequent projection 70b.

For completeness, Fig. 9 (bottom) shows the second projection ($n+1$) 70b and illustrates the shifted position 72b of the region of interest 72a selected in the first projection n 70a, as determined through the process described above. The shift is in fact not visible in this case, as it is on the order of less than one pixel (i.e. sub-pixel level). It is for this reason that the up-sampling of the two patches is valuable; this allows for sub-pixels shifts to be detected.

Another approach to determining a registration of the whole or part of the first and second attenuation patterns to one another is to make use of a trained machine learning algorithm such as a convolutional neural network (CNN) to predict the x-ray point source position differences.

In other words, it is proposed in some embodiments to determine the shift in the attenuation patterns between the two projections by a trained machine learning algorithm which is trained to receive as input the measurement data for the first and second projections and to generate as output a value of the shift between the 2D attenuation patterns of the first and second projections. The training data used in training the algorithm for example could be obtained from projections acquired from a dual point source CT system with a deliberately mis-calibrated system, where the distance between the two point sources is known in advance of acquiring the training data. The point source spacing could be iteratively adjusted and 2D attenuation patterns measured for multiple pairs of projections for each point-source spacing. The acquired attenuation pattern pairs for each spacing could be tagged with the known separation distance to which each corresponds (i.e. the ground truth). These could then be used in training a machine learning algorithm such as a CNN.

In accordance with one or more embodiments, a shift in the whole or part of the attenuation pattern (in the projection domain) can be determined indirectly based on first transferring the measurement data into the image domain and detecting a rotation in the image domain.

In particular, in accordance with one or more embodiments, the determining the shift in the whole or part of the attenuation patten of the first and second projection may be performed by: reconstructing a first image using only the x-ray projections among the received series of x-ray projections acquired with the first of the x-ray point sources; reconstructing a second image using only the x-ray projections among the received series of x-ray projections acquired with the second of the x-ray point sources; and detecting a rotation shift between the first image and the second image. More particularly, the first and second images may be axial (slice) images. The concept underlying this embodiment is that if the projection domain data is shifted translationally relative to the detector for each projection, then the resulting reconstructed (axial) image slice will have an overall rotation about the z-axis of the scanner.

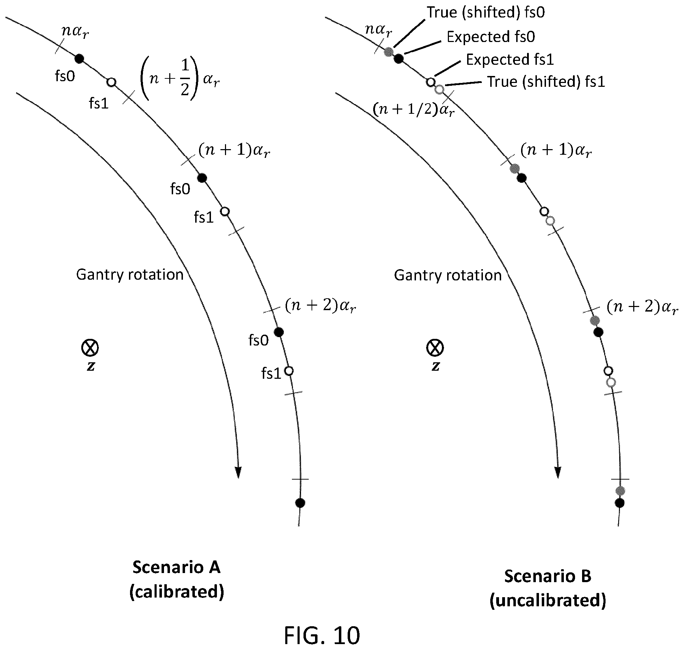

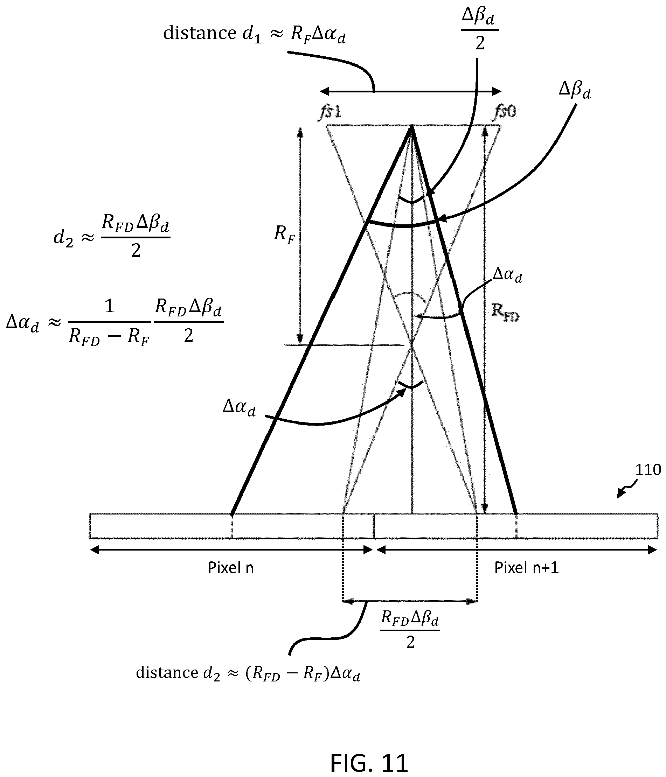

To illustrate this embodiment further, reference is now made to Fig. 10 and Fig. 11. Fig. 10 illustrates the positions of the two x-ray point sources (e.g. x-ray tube focal spots) fs0 and fs1 at different sampling points about the orbital rotational axis of the x-ray generator within the CT acquisition apparatus in a calibrated and uncalibrated scenario. Fig. 10 (left) shows a scenario ('Scenario A') in which the x-ray sources fs0 and fs1 are perfectly calibrated and thus their positions are as expected. Fig. 10 (right) shows a scenario ('Scenario B') in which the x-ray sources fs0 and fs1 have drifted from their expected positions. Thus, scenario B represents an uncalibrated scenario in which the true positions of the x-ray sources fs0 and fs1 have become shifted along the dimension of the orbital rotation axis. The sampling points for the first focal spot fs0 are shown with solid circles, and the sampling points for the second focal spot fs1 are shown with open circles.

Thus, within the axial reference frame, in effect the entire sampling pattern for fs0 is rotated counter-clockwise and for fs1 clockwise. When projected onto the detector, this appears as a translational shift in each projection along the direction of incrementing detector columns, i.e. the x-axis shown in Fig. 7. However, over the whole projection dataset for a single axial slice, this appears as a rotation about the z-axis in the dataset. The z-axis is in the direction into the page from the perspective of Fig. 10. Fig. 10 shows the z-axis direction using a cross-symbol. This indicates the z-direction but does not represent the true location of the z-axis, which would instead be at the center of rotation of the system.

As a result, if an image reconstruction is performed of just the fs0 data, then the resulting axial image is slightly rotated for the uncalibrated scenario (Fig. 10 right) as compared with the calibrated scenario (Fig. 10 left). The same holds true for a reconstruction using just the actual fs1 data, but the rotation direction is opposite. Thus, from the reconstructed images, it is possible to determine if the focal spot deflection or separation is correct.

Indeed, a quantitative analysis is possible. This can be understood further from Fig. 11. Fig. 11 shows the geometry of the dual point source (dual focal spot) system and illustrates the projection of the two point-sources fs0 and fs1 onto the detector array 110.

In this diagram, $R_{FD}$ = focus-to-detector distance (meaning the distance from the x-ray tube focal spot to the detector). $R_F$ is the focus-to-isocenter distance (meaning the distance from the x-ray tube focal spot to the imaging isocenter).

The angle $\Delta\alpha_d$ is the angular separation between the two focal spots fs1 and fs2 relative to the rotation isocenter. Using the small angle approximation (where $\tan(\Delta\alpha_d/2) \approx \Delta\alpha_d/2$, this corresponds to a spatial separation distance, $d_1$, between the two focal spots of approximately di = $R_F\Delta\alpha_d$

The angle $\Delta\beta_d$ is the angle between rays to (the respective center points) of neighboring detector pixels (detector elements). In this diagram, two neighboring detector pixels are shown, labelled n and *n+1.*

The projection of the respective rays from the first and second focal spots fs0, fs1 onto the detector is shown. Again, using the small angle approximation referred to above, the separation distance, $d_2$, between these ray projections on the detector is approximately equal to dz = $(R_{FD}-R_F)\Delta\alpha_d$. Thus a separation distance di between the focal spots fs0, fs1 can be mapped to a corresponding separation distance $d_2$ of the focal spot projections on the detector 110 by the relationship $d_1/d_2$ = $R_F/(R_{FD}-R_F)$.

According to the particular example system illustrated in Fig. 11, the target focal spot deflection distance $d_1 = R_F\Delta\alpha_d$ (i.e. the target distance between the two focal spots) is set according to the preferred constraint that the respective projected rays from the first (fs0) and second (fs1) focal spots through the iso-center are separated on the detector by a distance of

$$\text{d}_2 = \frac{R_{FD}\Delta\beta_d}{2}.$$

Thus, the target spacing $d_2$ between the projections of the two focal spots (or point sources) on the detector is, in this example, set approximately equal to

$$\text{d}_2 = \frac{R_{FD}\Delta\beta_d}{2}$$ and the angular separation between the rays from the two focal spots relative to the x-ray generator is $\Delta\beta_d/2$.

Using again the small angle approximation whereby $\Delta\alpha_d \approx \sin\Delta\alpha_d \approx \tan\Delta\alpha_d$, then the angular separation between the two focal spots relative to the rotation isocenter along the rotational axis of the generator is approximately equal to $$\Delta\alpha_d \approx \frac{1}{R_{FD}-R_F}\frac{R_{FD}\Delta\beta_d}{2}.$$

In operation, during imaging, the gantry is rotating with an assumed constant speed, and acquiring projections at an angular sampling rate of $\Delta\alpha_r$ (not shown in Fig. 11).

Following from the above, taking into account both the sampling rate and the focal spot separation, then the total focal spot deflection along the rotational axis of the generator for a single (first) projection would correspond to:

$$\alpha_{0,fs1} = \alpha_{0,fs0} - \frac{\Delta\beta_d}{2} \cdot \frac{R_{FD}}{R_{FD}-R_F} + \frac{\Delta\alpha_r}{2}$$

where $\alpha_{0,fs0}$ is the angular position of the first projection taken with fs0, $\alpha_{0,fs1}$ is the angular position of the first projection taken with fs1 and $\Delta\alpha_r$ is the angular sampling density of frames.

In the context of example embodiments of the present invention, it is proposed to detect a drift in the separation distance $d_1 \approx R_F\Delta\alpha_d$ between the focal spots fs0, fs 1 by detecting a relative rotation between a first image reconstructed using only the x-ray projections acquired with the first (fs0) of the x-ray point sources and a second image reconstructed using only the x-ray projections acquired with the second (fs1) of the x-ray point sources.

With reference to Fig. 10, here, in effect, the entire sampling pattern for fs0 is rotated by $\frac{\Delta\beta}{8}$ counter-clockwise and for fs 1 by the same angle clockwise.

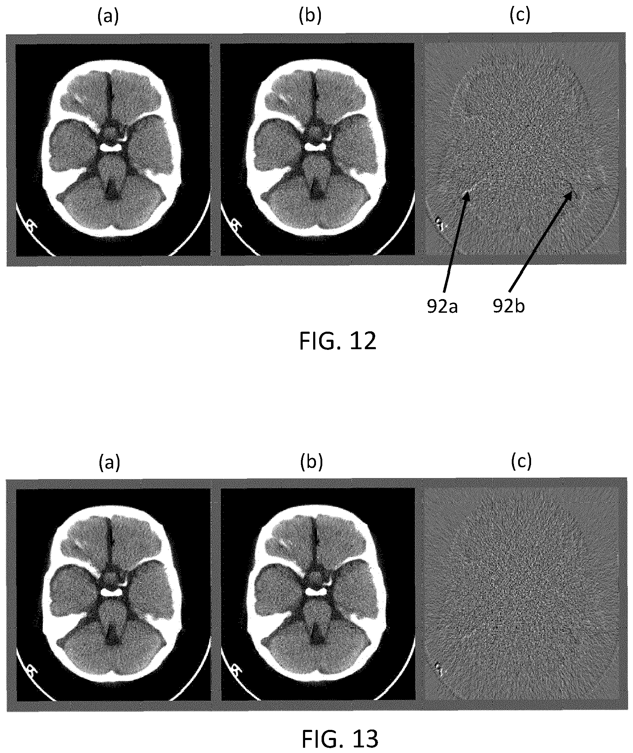

Fig. 12 and Fig. 13 illustrate the rotation effect in the image domain. Fig. 12 illustrates axial images for the scenario in which the focal spots are uncalibrated (scenario B in Fig. 10), and Fig. 13 illustrates axial images for the scenario in which the focal spots are calibrated (scenario A in Fig. 10). Fig. 12 (a) shows an axial image reconstructed using projections acquired only with fs0 and Fig. 12(b) shows an example axial image reconstructed using projections acquired only with fs1. In each of Fig. 12 and Fig. 13, image (c) shows a difference between image (a) and image (b).

It can be seen in the difference image of Fig. 12(c) that edges are visible (indicated by arrow 92a, 92b) which is characteristic of the two images (a) and (b) being slightly rotated with respect to each other. This indicates that the data acquired with fs0 and fs 1 are slightly inconsistent.

By contrast, in the difference image of Fig. 13(c), the edges do not show up as strongly, indicating less rotation between the two images.

As mentioned above, in operation, a first image reconstructed with only fs0 data would be compared with a second image reconstructed with only fs1 data and a relative rotation between the two detected, e.g. through forming a difference image or by performing a rotation registration between the two. The detected rotation is quantified by a rotation metric, e.g. degrees of rotation or a more indirect metric of rotation. This is then compared against an expectation for the relative rotation to see if the relative rotation falls within an expected range of values for the rotation metric. The expectation range would depend typically upon how the reconstruction algorithm(s) are configured. When generating the example images of Fig. 12 and Fig. 13, respective reconstruction algorithms were employed which were configured according to an expected positioning of the focal spots corresponding to the calibrated positions. For this reason, the images reconstructed from the projection data acquired in the calibrated state shows almost no rotation in the difference image, because the true focal spot positions match the expected positions. Thus, in this case, the expectation range for the rotation between the first and second image might be a range centered about a zero value, since, if the focal spots are properly calibrated, the reconstructions algorithms are configured such that the resulting first and second images should exactly rotationally align. However, this is not essential. In other cases, the reconstruction algorithm could be differently configured meaning that the expectation range for the rotation between the first and second image in the event of perfect calibration may be non-zero.

In accordance with any of the embodiments of the invention, the method may be improved by performing an initial pre-processing of the measurement data corresponding to each projection in order to re-bin the data into a parallel beam geometry. In preferred examples, wedge-beam rebinning is performed. In wedge rebinning, the cone-beam is transformed into a wedge-shaped beam. Wedge beam rebinning is described for example in section II.A of the paper: Shechter et al.: The frequency split method for helical cone-beam reconstruction. Med Phys. 2004 Aug;31(8):2230-6.

Parallel beam rebinning of this kind adds some additional computational cost, but may improve robustness. This can be understood from the fact that embodiments of the invention are based on detecting a shift in the detected attenuation pattern at the detector between the first and second x-ray sources. The attenuation pattern is naturally a projection of the attenuation distribution of the scanned object. Furthermore, the center of attenuation of the measured attenuation pattern at the detector is actually the projection of the center of attenuation of the object projected to the (parallel beam) detector. Since the center of mass of the imaged object is usually close to the iso-center of the system, it barely moves once being projected onto the parallel beam detector. It is for this reason that the parallel beam geometry is useful and increases robustness.

According to any of the embodiments of the present invention, it may be advantageous before beginning the calibration check to initially perform an object alignment check, to check that the imaged object is fully contained within the projection span, and is not truncated. Such a test is in fact typically already integrated into many modern CT systems.

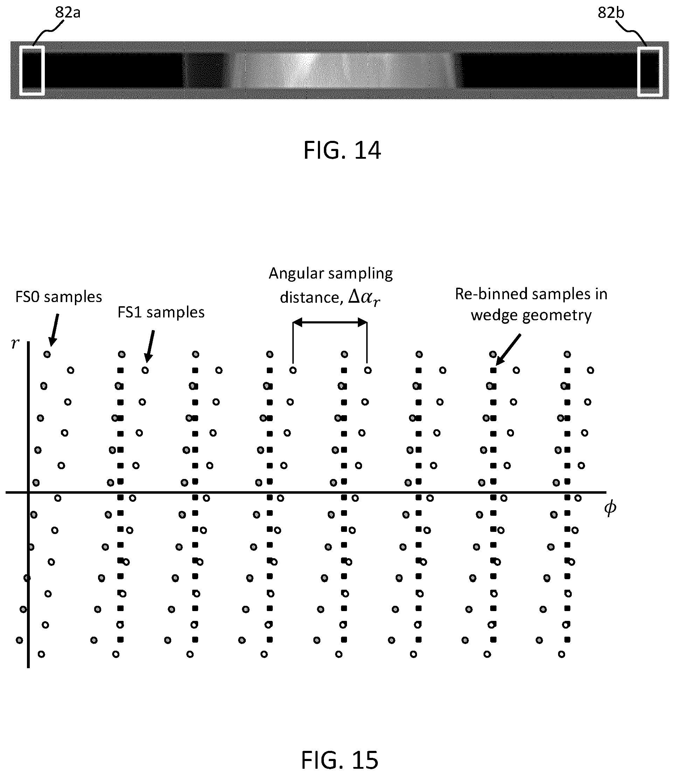

With reference to Fig. 14, the process for checking object alignment (i.e. non-truncation) for a given projection may comprise the following steps.

The process may comprise identifying the measured attenuation values in the measured 2D attenuation pattern for the projection corresponding to a pre-defined boundary region of the array of detector elements. Fig. 14 illustrates with boxes 82a, 82b an example boundary region of an example measured projection attenuation pattern. The boundary region may be defined for example as corresponding to the attenuation pattern region formed by a pre-defined number of detectors columns on each far side of the 2D attenuation pattern, i.e. the outermost detector columns.

The process may further comprise determining whether each of the measured attenuation values in the boundary region 82a, 82b fall within a pre-defined null range, the null range corresponding to a zero-attenuation measurement. In other words, it is checked whether the boundary region is empty. If it is not empty then this potentially indicates that the object is truncated in the projection and spills over the side of the projection boundary. The process may further comprise, responsive to determining that not all of the measured attenuation values in the boundary region 82a, 82b fall within the null range, generating an alert message and/or aborting the calibration check.

In some embodiments, the method may further comprise computing an actual distance between the first and second x-ray point sources based on a determined translation in the whole or part of the attenuation pattern of the first and second consecutive projections. This may be based on a pre-formed lookup table or transfer function which permits mapping from the computed shift in the whole or part of the attenuation patterns and the separation distance between the two point sources. This lookup table or transfer function can be determined in advance empirically through a simple calibration operation, or a machine learning algorithm could be trained to output a value of the separation distance, as has already been described above.

Indeed, the separation distance can be computed using an equation or function if the geometry of the system is known. In particular, with reference again to Fig. 11, using the small angle approximation, the separation distance di between the two point sources fs0, fs1 along the axis of the generator rotation is approximately equal to $d_1 \approx R_F \Delta\alpha_d$, where $\Delta\alpha_d$ is the angular separation between the two focal spots fs 1 and fs2 relative to the rotation isocenter, and $R_F$ the focus-to-isocenter distance (meaning the distance from the x-ray tube focal spot to the imaging isocenter). The distance, $d_2$, between the ray projections of fs0 and fs1 on the detector 110 is approximately equal to $d_2 \approx (R_{FD}-R_F)\Delta\alpha_d$. Thus, by simple substitution for $\Delta\alpha_d$, one can convert a detected distance shift, $d_2$, at the detector 110 (i.e. a change in $(R_{FD}-R_F)\Delta\alpha_d$) to a corresponding shift in the separation distance, $d_1$, between the focal spots along the rotational axis of the generator (i.e. corresponding change in $R_F\Delta\alpha_d$). For example, $d_1/d_2 = R_F/(R_{FD}-R_F)$.

Here (with reference to Fig. 11): $R_{FD}$ = focus-to-detector distance (meaning the distance from the x-ray tube focal spot to the detector); $R_F$ is the focus-to-isocenter distance (meaning the distance from the x-ray tube focal spot to the imaging isocenter).

According to some embodiments, a response action may be performed following the performance of the calibration check.

Some embodiments propose to perform a correction.

In particular, the method may comprise determining a deviation in the distance between the first and second x-ray point sources and a target distance between the first and second x-ray point sources. The method may further comprise communicating with an image reconstruction module to apply an additional correction step to a reconstruction algorithm comprised by the reconstruction module based on the determined deviation. Optionally, the method may further comprise supplying the CT data to the reconstruction module and applying the reconstruction algorithm with the correction to the CT data.

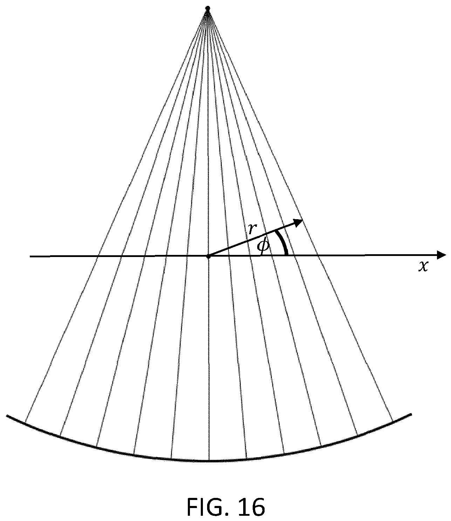

By way of further clarification of some of the concepts discussed earlier, Fig. 15 illustrates how the CT apparatus samples line integrals using the standard $r\phi$ diagram. For further clarity, Fig. 16 illustrates the mapping of measured rays into r, $\phi$ space. Each measured (intensity or attenuation) value or sample (measured at the detector) is represented by a dot in the diagram of Fig. 15.

Referring to Fig. 15, each sample is assumed to correspond to a projection of a single straight-line ray from the source to the point on the detector at which the measurement was taken. With reference to Fig. 16, the value $r$ corresponds to the distance of this ray to the rotation iso-center. The value $\phi$ corresponds to the angle of this ray to the x-axis, where the x-axis is the cartesian x-axis of the plane defined by the fan of rays. Within this geometry, the previously referenced angular sampling interval, $\Delta\alpha_r$, (often called radial sampling density or just 'radial sampling') is defined as the difference of the radial values $r$ of neighboring detector measurements, as measured at the isocenter of rotation.

All dots from a single projection lie on a slanted line. For subsequent projections, the dots are shifted by an amount equal to the angular sampling interval. Fig. 15 indicates with arrows for example, the slanted line of measurements corresponding to the first projection in this diagram for first point source fs0 and the slanted line of measurements corresponding to the first projection in this diagram for second point source fs1. Samples acquired with point source fs0 are shown as solid grey dots. Samples acquires with fs1 are shown as white dots. Fig. 15 further illustrates the angular sampling interval $\Delta\alpha_r$ which corresponds to the separation distance between the two point-sources fs0, fs1.

As explained above, the raw samples are preferably re-binned to parallel beam geometry, and preferably wedge beam geometry. The straight vertical lines of samples in Fig. 15 indicate the re-binned sample points. The re-binning may involve interpolating from the raw measurement points to the re-binned points. The re-binned points are shown as black squares.

A reconstruction algorithm is configured to receive as input a set of attenuation measurements from the detector array for each projection, from the alternating point sources. Each attenuation measurement may be labelled or tagged as corresponding to a measurement point in r, $\phi$ space. This may involve mapping the measurement points from each projection from the detector array geometry to corresponding ray geometry in r, $\phi$ space.

The drift in the separation distance between the x-ray point sources leads to a shift in the $r$ co-ordinates of the measurements of each projection. The measurement points for each fs0 projection are shifted slightly upwards and the measurement points for each fs 1 projection are shifted slightly downwards. At the same time, points are shifted slightly to the right and left, respectively, see Fig. 2.

To implement a correction in the reconstruction therefore, this may be implemented by applying a correction to the measurement data before the reconstruction algorithm is applied. In particular, to compensate for a shift in the separation between the point sources, this may comprise adjusting the $r$ and $\phi$ co-ordinates of the measurement points for at least one of the two point sources by an interval amount equal to the total detected change in the separation distance between the two focal points.

With respect to the correction, it is noted that the appearance of aliasing artefacts and loss of resolution for improperly calibrated focal spot positions has two causes. The first cause is that the actual focal spot spatial positions do not match the theoretically optimal positions. This part is not corrected by the above procedure. However, the second cause is that the reconstruction algorithm assumes that the actual positions match the optimal positions. Knowledge of the actual positions therefore allows for a correction to be made to compensate for the change in geometry, and thereby minimize the negative impact of the miscalibration.

In some embodiments, the method may further comprise generating a status report indicative of a difference between the computed distance between the first and second x-ray point sources and a pre-defined target distance between the first and second x-ray point sources, and transmitting the status report to a server computer.

In some embodiments, the system further includes a data log, and the method further comprises storing the measured distance between the first and second x-ray point sources in the data log. In this way, the system automatically logs information about the estimated focal spot positions and may raise a red flag if the positions are outside of acceptable bounds.

In some embodiments, the method may further comprise generating an alert signal for communication to a user interface or a further computing device responsive to said determined shift being outside of said expected range.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A computer-implemented method (10) for determining calibration status of x-ray tube point sources within a dual point source computed tomography (CT) acquisition apparatus, the method comprising:

    receiving (12) dual point source CT data comprising x-ray detector measurement data for each of a series of x-ray projections, the measurement data corresponding to measurement data acquired with an x-ray generator of a CT acquisition apparatus providing alternately activated first and second x-ray point sources, the point sources displaced relative to one another along an orbital rotational axis of the generator within the CT acquisition apparatus, and wherein the measurement data includes for each x-ray projection a 2D attenuation pattern corresponding to measured attenuation values for a 2D array of detector elements;
perform (14) a point source calibration check, comprising:

> determining (20), using the measurement data, a shift of the whole or part of the attenuation pattern of a first and second consecutive projection, each of the first and second projection generated using a different one of the two point sources; and
> determining (22) whether the shift is within a pre-defined expected range.

2. The method of claim 1 wherein determining the shift comprises determining a translation in said direction of the orbital rotation axis of the generator.

3. The method of claim 1 or 2, wherein determining the shift comprises identifying a characteristic point in the respective 2D attenuation patterns of the first and second projections and determining a translation in said characteristic point between the first and second projections.

4. The method of claim 3, wherein the characteristic point is defined in each 2D attenuation pattern based on a function of attenuation values in the attenuation pattern, weighted by intensity value position in the 2D pattern.

5. The method of claim 4, wherein the characteristic point is defined as a center of attenuation along at least one dimension of each 2D attenuation pattern, the center of attenuation being an attenuation analog of center of mass.

6. The method of claim 5, wherein the center of attenuation along at least one dimension of the attenuation pattern is defined by the equation:

$$c = \frac{\sum_j j * p'(j)}{\sum_j p'(j)}$$

where $p'(j) = \Sigma_i p(i,j)$,

where $i$ is an index of the detector element row and $j$ is an index of the detector element column, and
where $p(i,j)$ is the measurement value for detector element in row $i$, column $j$.

7. The method of claim 1 or 2, wherein determining the shift comprises identifying a characteristic sub-region of the 2D attenuation pattern of each of the first and second projections, and performing a translational registration between the characteristic sub-regions of the two projections.

8. The method of any of claims 1-7, further comprising, in advance of the calibration check, performing, for each projection, an imaged object alignment check comprising:

> identifying the measured attenuation values in the 2D attenuation pattern corresponding to a pre-defined boundary region of the array of detector elements;
> determining whether each of the measured attenuation values in the boundary region fall within a pre-defined null range, the null range corresponding to a zero-attenuation measurement; and
> responsive to determining that not all of the measured attenuation values in the boundary region fall within the null range, generating an alert message and/or aborting the calibration check.

9. The method of any of claims 1-8, wherein determining the shift comprises using a trained machine learning algorithm which is trained to receive as input the measurement data for the first and second projections and to generate as output a value of the shift between the 2D attenuation patterns of the first and second projections.

10. The method of any of claims 1-9, wherein the determining the shift in the whole or part of the attenuation patten of the first and second projection is performed by:

> reconstructing a first image using only the x-ray projections among the received series of x-ray projections acquired with the first of the x-ray point sources;
> reconstructing a second image using only the x-ray projections among the received series of x-ray projections acquired with the second of the x-ray point sources; and
> detecting a rotation between the first image and the second image.

11. The method of any of claims 1-10, further comprising computing an actual distance between the first and second x-ray point sources based on a determined translation or rotation in the whole or part of the attenuation pattern of the first and second consecutive projections.

12. A computer-implemented method (10, 118) for reconstructing CT image data from a dual point source computed tomography (CT) acquisition apparatus having acquired measurement data with an x-ray generator providing alternately activated first and second x-ray point sources, the point sources displaced relative to one another along an orbital rotational axis of the generator within the CT acquisition apparatus, and wherein the measurement data includes for each x-ray projection a 2D attenuation pattern corresponding to measured attenuation values for a 2D array of detector elements, the method comprising:

   obtaining a distance estimate between the first and second x-ray point sources; and
   reconstructing CT image data based on the distance estimate.

13. A computer program product comprising computer program code configured, when run on a processor, to cause the processor to perform a method in accordance with any of claims 1-12.

14. A processing device (32) comprising:

   an input/output (34); and
   one or more processors (36) configured to perform a method, the method comprising:
   receiving, at the input/output, dual point source CT data comprising x-ray detector measurement data (42) for each of a series of x-ray projections, the measurement data corresponding to measurement data acquired with an x-ray generator of a CT acquisition apparatus (40) providing alternately activated first and second x-ray point sources, the point sources displaced relative to one another along an orbital rotational axis of the generator within the CT acquisition apparatus, and wherein the measurement data includes for each x-ray projection a 2D attenuation pattern corresponding to measured attenuation values for a 2D array of detector elements;
   perform a point source calibration check, comprising:

      determining, using the measurement data, a shift of the whole or part of the attenuation pattern of a first and second consecutive projection, each of the first and second projection generated using a different one of the two point sources; and
determining whether the shift is within a predefined expected range.

15. A system (30), comprising:

   a dual point source CT acquisition apparatus (40); and
   a processing device (32) as claimed in claim 14.

FIG. 1

FIG. 2

FIG. 3

48a
fs1
fs2
fs0
48b

FIG. 4

10

| Receive dual source CT image data | 12 |

14

Detect shift in intensity patten between 1$^{st}$ and 2$^{nd}$ source — 20

Compare shift against expectation — 22

FIG. 5

32
42
CT apparatus — 40

34
I/O

38
Memory

30

proc — 36

FIG. 6

FIG. 7

FIG. 8

Projection $n$

Projection $n + 1$

FIG. 9

$n\alpha_r$

fs0

fs1

$\left(n + \dfrac{1}{2}\right)\alpha_r$

$(n + 1)\alpha_r$

fs0

fs1

Gantry rotation

$\bigotimes$
$z$

$(n + 2)\alpha_r$

fs0

fs1

**Scenario A
(calibrated)**

True (shifted) fs0

Expected fs0

Expected fs1

True (shifted) fs1

$n\alpha_r$

$(n + 1/2)\alpha_r$

$(n + 1)\alpha_r$

Gantry rotation

$\bigotimes$
$z$

$(n + 2)\alpha_r$

**Scenario B
(uncalibrated)**

FIG. 10

$$\text{distance } d_1 \approx R_F \Delta\alpha_d$$

$$\frac{\Delta\beta_d}{2}$$

$$\Delta\beta_d$$

$$d_2 \approx \frac{R_{FD}\Delta\beta_d}{2}$$

$$\Delta\alpha_d \approx \frac{1}{R_{FD} - R_F}\frac{R_{FD}\Delta\beta_d}{2}$$

$$R_F$$

$$\Delta\alpha_d$$

$$R_{FD}$$

$$\Delta\alpha_d$$

$$fs1 \qquad fs0$$

110

Pixel n

Pixel n+1

$$\frac{R_{FD}\Delta\beta_d}{2}$$

$$\text{distance } d_2 \approx (R_{FD} - R_F)\Delta\alpha_d$$

FIG. 11

(a)       (b)       (c)

92a   92b

FIG. 12

(a)       (b)       (c)

FIG. 13

FIG. 14

FIG. 15

FIG. 16

Europäisches
Patentamt

European
Patent Office

Office européen
des brevets

# EUROPEAN SEARCH REPORT

Application Number

EP 22 21 4097

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/020921 A1 (DONG FANG FRANK [US] ET AL) 28 January 2010 (2010-01-28) | 1-3,7-9, 11-15 | INV. A61B6/03 A61B6/00 |
| A | * paragraphs [0031], [0032], [0043], [0036] * | 4-6,10 | |
| A | US 2019/059846 A1 (JIANG YIFENG [CN]) 28 February 2019 (2019-02-28) * the whole document * | 4-6,10 | |
| A | WO 2019/095838 A1 (SHENZHEN INST ADV TECH [CN]) 23 May 2019 (2019-05-23) * the whole document * | 4-6,10 | |

TECHNICAL FIELDS
SEARCHED (IPC)

A61B

The present search report has been drawn up for all claims

| Place of search | Date of completion of the search | Examiner |
|---|---|---|
| Munich | 2 May 2023 | Anscombe, Marcel |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
................................................................
& : member of the same patent family, corresponding
   document

EPO FORM 1503 03.82 (P04C01)

## ANNEX TO THE EUROPEAN SEARCH REPORT
### ON EUROPEAN PATENT APPLICATION NO.

EP 22 21 4097

This annex lists the patent family members relating to the patent documents cited in the above-mentioned European search report.
The members are as contained in the European Patent Office EDP file on
The European Patent Office is in no way liable for these particulars which are merely given for the purpose of information.

02-05-2023

| Patent document cited in search report | Publication date | Patent family member(s) | | Publication date |
|---|---|---|---|---|
| US 2010020921 A1 | 28-01-2010 | NONE | | |
| US 2019059846 A1 | 28-02-2019 | CN | 108670284 A | 19-10-2018 |
| | | EP | 3675741 A1 | 08-07-2020 |
| | | US | 2019059846 A1 | 28-02-2019 |
| | | US | 2020289066 A1 | 17-09-2020 |
| | | WO | 2019041223 A1 | 07-03-2019 |
| WO 2019095838 A1 | 23-05-2019 | CN | 108030501 A | 15-05-2018 |
| | | WO | 2019095838 A1 | 23-05-2019 |

EPO FORM P0459

For more details about this annex : see Official Journal of the European Patent Office, No. 12/82

**EP 4 385 417 A1**

## REFERENCES CITED IN THE DESCRIPTION

### Patent documents cited in the description

- US 4637040 A

### Non-patent literature cited in the description

- **M. KACHELRIESS et al.** Flying Focal Spot (FFS) in Cone-Beam CT. *IEEE TNS,* 2006, vol. 53 (3

- **SHECHTER et al.** The frequency split method for helical cone-beam reconstruction. *Med Phys,* August 2004, vol. 31 (8), 2230-6